# EUROPEAN PATENT APPLICATION

(11) **EP 1 568 316 A1**
(43) Date of publication of application: **31.08.2005**
(21) Application number: 04004114.7
(22) Date of filing: 24.02.2004
(51) Int. Cl.: A61B 5/024

(54) **Assessment of fetal reactivity by fetal heart rate analysis**

(71) Applicant: Neoventa medical AB, 431 35 Mölndal (SE)
(72) Inventor: Outram, Nicolas, Tavistock PL19 8DB (GB); Rosén, Karl Gustaf, 442 35 Kungälv (SE)
(74) Representative: Lind, Urban Arvid Oskar

(57) **Abstract**

The present invention relates to the field of fetal heart rate (FHR) monitoring. In particular, it relates to monitoring during periods of so-called non-stationary fetal heart rate patterns, such as those that occur during labour.

The invention provides an improved apparatus, method and computer program for more clear analysis of FHR variations, such that fetal distress can be correctly assessed earlier.

According to one aspect, the invention comprises means for determining a fetal heart rate, means for identifying a primary fetal heart rate component, means for subtracting the primary component from the determined fetal heart rate to determine a residual component, and means for using said residual component to estimate the fetal heart rate beat-to-beat variation.

## Description

### TECHNICAL FIELD OF THE INVENTION

The present invention relates to the field of fetal heart rate (FHR) monitoring. In particular, it relates to monitoring during periods of so-called non-stationary fetal heart rate patterns, such as those that occur during labour.

### BACKGROUND

The fetal heart rate is an important indicator of fetal health during pregnancy and labour. Some variability in the heart rate is desirable, as this shows that the fetus is responding to stimuli. However, certain changes can also indicate that the fetus is experiencing distress and may be suffering from the early stages of hypoxia (lack of oxygen).

It is common practice for the FHR to be measured during labour. At present, the FHR is normally presented as a plot against time on a monitor. It is then studied visually by medical personnel who check for signs of fetal distress. Thus, the system relies upon the skill and experience of the user.

During gestation and birth the fetus alternates between sleep states and active states and these are indicated by a change in FHR. These changes occur suddenly and each state lasts for a period of up to 60 minutes, depending on the development of the fetus. An experienced obstetrician is able to observe these alterations and determine whether these are normal for the stage and development of the fetus.

During labour, the fetus is subject to additional influences, such as uterine contractions, that may significantly alter its FHR by means of intermittent umbilical cord compression, mechanical pressure etc. In addition, other factors which affect the heart rate, such as oxygenation, drugs, fetal breathing activity, glucose levels etc, can vary significantly during labour in a manner which is not witnessed previously during pregnancy. This results in comparatively long term changes in the FHR, as opposed to the abrupt shifts caused by a change in state, and creates what is called a non-stationary heart rate.

These changes mean that a non-stationary heart rate is much harder to decipher, even for an experienced obstetrician. Often, early warning signs provided by anomalies in the FHR beat-to-beat variation are misinterpreted or missed altogether. This can result in severe intrapartum hypoxia. Alternatively, upon viewing an abnormal FHR reading, these (normal) changes may cause obstetricians to err on the side of caution which results in unnecessary surgical intervention.

In order to assist in the correct identification of fetal distress, the present applicants have developed the STAN® fetal monitoring system. Its methodology is based upon the combination of FHR monitoring and ST waveform analysis of the fetal ECG. The system automatically detects abnormalities in the ST waveform (called ST events) that are indicative of hypoxia. The ST events are in effect used as an alarm to allow the operator to focus on the FHR patterns.

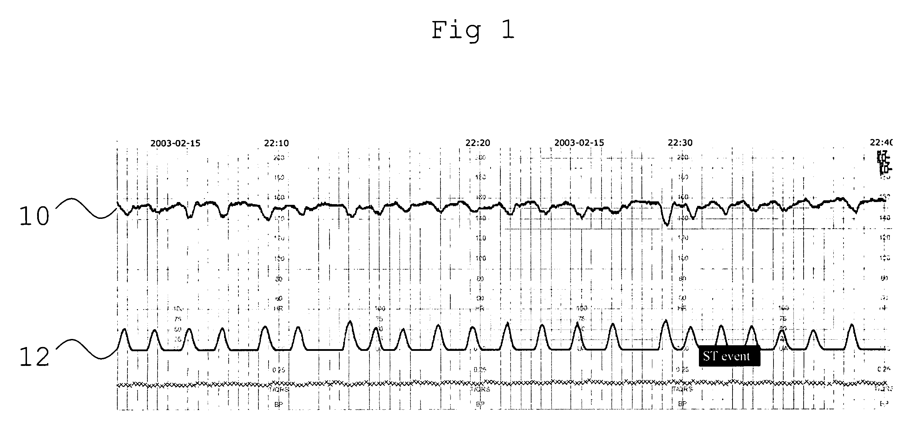

Potential problems with the fetus are indicated by extremes in FHR variation. FIG 1 shows a 35 minute STAN® recording in a 1^{st} stage of labour in which the fetus is exposed to slowly developing hypoxia. The recording shows the FHR 10 together with the uterine activity 12. Here, the fetus is showing signs of reduced beat-to-beat variation and no reactivity (i.e. no response to stimuli). Although such a pattern can normally be identified visually, there are situations where this may be missed if no other warnings are given by additional medical equipment. Further, when the FHR variability gradually decreases over time, these slight alterations may be overlooked.

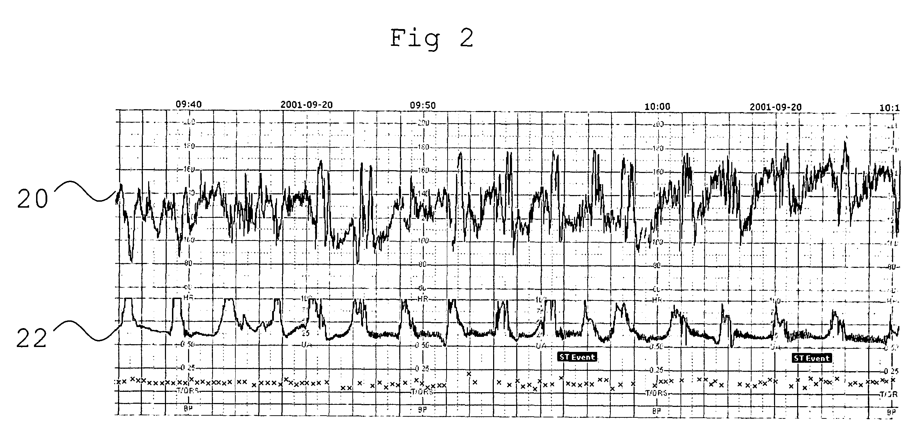

FIG 2 shows an incidence of increased FHR variation. Again, the FHR 20 is shown together with uterine activity 22. This recording was taken during the last stages of labour when is it normal for large variations in FHR to occur. However, in this instance the increased variation was a response to developing hypoxia. In situations such as these, it is not uncommon for the FHR to be misread as normal, or at least ambiguous. This can lead to delays which may severely affect the health of the baby.

There therefore exists a need for a method of more clearly analysing FHR variations, in particular during labour, such that fetal distress (hypoxia) can be correctly diagnosed earlier.

Previously, attempts have been made to quantify FHR variation by standard statistics (analysis of variance, standard deviation etc) and more sophisticated power spectrum analysis. Several studies have been conducted on spectral analysis of the FHR as a method to quantify changes over time in FHR variation. One such study is discussed in "Quantification of the fetal heart rate variability by spectral analysis of fetal well being and fetal distress", Akselrod S et al, Eur J Obstet Gynecol Reprod Biol 1994; 54; 103-8. However, spectral analysis requires a continuous and stationary sequence of FHR data and does therefore not lend itself to continuous assessment of FHR during labour, when the FHR tends to be non-stationary. Consequently, these methods have been of little use during labour.

Computerised analysis of FHR variation has also been applied to FHR tracings obtained prior to the onset of labour. Sonicaid 8000 is a system currently being marketed to assist in the quantification of FHR variation before labour starts ("Antenatal cardiotocogram quality and interpretation using computers" Dawes GS et al, Br J Obstet Gynaecol. 1992 Oct; 99(10): 791-7). This system is based on applying standard techniques of FHR beat-to-beat variance measurements (mean values, standard error of the mean etc) and again does not lend itself to continuous FHR variation assessments in a non-stationary FHR signal. Thus, computerised assessment of FHR beat-to-beat variations has not proved helpful during labour.

Consequently, FHR variation during labour is still conducted visually by a skilled, experienced medical practitioner, which can occasionally lead to signs of fetal distress being overlooked or wrongly diagnosed, as discussed above.

### SUMMARY OF THE INVENTION

It is therefore an object of the present invention to mitigate limitations related to the background art described above.

A further object of the present invention is to provide an improved apparatus, method and computer program for more clear analysis of FHR variations, in particular during labour, such that fetal distress (hypoxia) can be correctly diagnosed earlier.

Another object of the present invention is to provide an improved computerised assessment of FHR variations during labour.

According to a first aspect of the present invention, there is provided an apparatus for fetal monitoring comprising:
means for determining a fetal heart rate,
means for identifying a primary fetal heart rate component which is required to shift a volume of blood from the heart to the cardiovascular system, and
means for subtracting the primary component from the determined fetal heart rate to determine a residual component; and
means for using said residual component to estimate the fetal heart rate beat-to-beat variation.

The invention is based upon recognition by the inventors that the FHR can be separated into two elements. Firstly, there is the FHR component which is required to allow the heart to serve as a pump and shift a volume of blood to the cardiovascular system. This is defined as the primary FHR component. As well as this basic requirement, as previously discussed, the FHR is under the influence of numerous other sources. These secondary FHR components, most of which are under the influence of higher central nervous system structures, are termed the FHR residual component and comprise the remainder of the FHR.

It is this FHR residual component which provides a measure of external influences and, most importantly, the ability of the central nervous system to react and respond to changes in the internal milieu not primarily associated with the need to shift blood volume. By identifying this, the invention allows indications of fetal distress to be more readily identified.

The present invention also extends to a corresponding method and therefore viewed from another aspect the invention provides an method for fetal monitoring comprising:
means for determining a fetal heart rate,
means for identifying a primary fetal heart rate component which is required to shift a volume of blood from the heart to the cardiovascular system, and
means for subtracting the primary component from the determined fetal heart rate to determine a residual component; and
means for using said residual component to estimate the fetal heart rate beat-to-beat variation.

The FHR residual component, r, can be isolated by identifying the primary FHR component, p, and subsequently subtracting this from the recorded beat-to-beat variation, y, leaving only the FHR residual. This can be expressed mathematically by the equation r = y - p.

The FHR residual component can then be analysed to provide information on the reactivity of the fetus and thus its health. This may be done visually, for example by studying a plot on a monitor, but preferably statistical tests are applied. For example, the frequency distribution of residual measurements, median, 95^{th} percentile, etc may be determined.

In order to obtain useful results, it is clearly important to correctly identify the FHR primary component. Several techniques can be used to achieve this, such as integration of the recorded data over a predetermined time interval. However, it has been found that it is preferable to identify the primary FHR component through a polynomial curve fit approximation of the recorded data.

This is particularly useful during labour when the non-stationary nature of the FHR makes other methods, such as integration, unreliable. In these circumstances the approximation should preferably be instantaneous and therefore a polynomial curve fit provides the best results. Furthermore, a polynomial curve fit would allow for the primary FHR component to be calculated despite intermittent loss of FHR data, a constant feature during the final stage of delivery.

Through experimentation with previously recorded FHR variation data, it has been found that it is preferable to use a least squares polynomial approximation. It is most preferable that the polynomial is a 5^{th} order polynomial approximation.

A least squares approximation may be formulated by minimising the function |**P**x-y|², where y is a vector representative of n heart rate samples, **P** is an NxM matrix where the columns are discrete independent polynomial functions and x is a vector of real valued coefficients. The well-known least squares solution for the coefficient vector is x=(**P**^{T}**P**)⁻¹**P**^{T}y. The approximation of the primary FHR component, p, will therefore be the projection of y onto the subspace spanned by **P**, given by p=**P**x=**P**(**P**^{T}**P**)⁻¹**P**^{T}y. The success of this projection, for any given application, depends on how much the desired solution space is spanned by the subspace defined by **P**.

However, making the best choice of **P** can be difficult for some functions, in particular in the case of biomedical signals such as the heart rate as there is no *a priori* information on the nature of the function. It is known that the heart rate has regions of high and low frequencies within any interval of interest. Such a situation can lead to a "ringing" phenomenon when using polynomial approximations, thus causing inaccurate results.

One preferred solution is to divide the data into small adjacent regions and perform independent polynomial approximations in each region. This improves the accuracy of the result as there are then a guaranteed maximum number of degrees of freedom (turning points) in each region. Furthermore, as each approximation is independent of the others, they will not induce "ringing".

As each approximation is independent, the polynomial curves may not align at the region boundaries. To overcome this and achieve a continuous function, it is preferable to apply constraints to the formulation of each approximation. These constraints are preferably that neighbouring polynomial functions must align and have equal first derivatives (i.e. gradients) at the region border where they join. The constraints may be applied using the well-known method of Lagrange multipliers. It can be shown that by formulating these constraints with known polynomials, e.g. Legendre or Chebyshev, new discrete and independent polynomials can be derived that more closely span the desired solution space.

Viewed from another aspect therefore, the present invention provides a method for determining the primary FHR component in a recording of fetal heart rate beat to beat variation, comprising the steps of dividing the recording into regions of a predetermined size and performing individual polynomial approximations in each region, wherein each polynomial approximation is constrained such that neighbouring polynomial functions align and have equal first derivatives at the region border where they join.

By visual inspection and empirical testing, it has been found that an excellent primary FHR variation approximation can be achieved through applying consecutive constrained 5^{th} order Legendre polynomial approximations, each spanning 20 FHR samples.

As mentioned above, once the FHR residual component has been isolated from the primary FHR component, it can be analysed statistically.

One such method is to monitor the 95^{th} percentile recorded over specified time blocks. It has been found that consistent readings of a running 20 minutes median value of <3ms indicate a low FHR variation giving rise to concern for the wellbeing of the fetus.

Further, the residual frequency distribution in the 3-4ms domain has been identified as the highest band most consistently containing FHR residuals regardless of FHR variation. Thus is it preferable to monitor the frequency distribution in this range. It has been found that a frequency distribution within the 3-4ms domain with periods of >7% indicates a healthy level of reactivity. This function is based on the observation that a fetus, although in a sleep state with low beat-to-beat variation, will express intermittent bursts of activity as detected by an increase in instantaneous beat-to-beat variation. The conventional method of assessing reactivity features is to visually identify episodic accelerations. This is believed to be inventive in itself and so, viewed from another aspect, the invention provides a method of indicating the possibility of fetal distress comprising the steps of: obtaining FHR residual component data, providing a reading of the 95^{th} percentile and providing a reading of the 3-4ms frequency distribution. Table I gives the characteristics of the residual measurements required to assess a normal, healthy fetus with a reactive FHR trace, a fetus unable to respond to the stress of labour (Preterminal) as well as the fetus forced to respond with an extraordinary regulatory effort (Alarm reaction).

In Table I, FD₃₋₄ₘₛ means the frequency distribution of FHR residual measurements recorded within the 3-4ms domain, and 95^{th} 20' median means the 95^{th} percentile of residual measurements recorded as a running 20 minutes median value.

**Table I:**

| Criteria to indicate preterminal FHR pattern and alarm reaction from FHR residual measurements. | | | |
|---|---|---|---|
| Residual measurements | Observations made during the first 30 minutes of a recording | | |
| | Normal at onset | Ambiguous at onset | Preterminal status at onset |
| | FD₃₋₄ₘₛ consistently >3% or 1-3% range but with episodes of >7.0% | All other observations. Accelerations will indicate normality. | FD₃₋₄ₘₛ consistently <1% in a 10' block of data or consistently <2% during the 30 minutes |
| | Observations made after the first 30 minutes of a recording to indicate a preterminal fetal heart rate pattern | | |
| Residual measurements | 95^{th} 20' median consistently below 3 ms for 90 min | 95^{th} 20' median consistently below 3 ms for 60 min | 95^{th} 20' median consistently below 3 ms for 60 min |
| | 95^{th} 20' median consistently below 3 ms for 40' and FD₃₋₄ₘₛ consistently <1% in a 20' block of data | 95^{th} 20' median consistently below 3 ms for 40' and FD₃₋₄ₘₛ consistently <1% in a 20' block of data | 95^{th} 20' median consistently below 3 ms for 20' and FD₃₋₄ₘₛ consistently <1% in a 20' block of data |
| | | Note: FD_{3-4 raw} >7.0% for >2' will automatically transfer the case to a Normal status. | |
| | Observations to indicate an Alarm reaction | | |
| Residual measurements | 95^{th} 20' median > 25 ms for > 6 minutes | | |

Preferably the FHR residual data is provided as discussed above. The invention also extends to a monitoring apparatus arranged to perform this method.

According to another aspect of the present invention it extends to a computer program for executing the steps of:
determining a fetal heart rate,
identifying a primary fetal heart rate component which is required to shift a volume of blood from the heart to the cardiovascular system, and
subtracting the primary component from the determined fetal heart rate to determine a residual component; and
using said residual component to estimate the fetal heart rate beat-to-beat variation.

### BRIEF DESCRIPTION OF THE DRAWINGS

An embodiment of the invention will now be described, by way of example only, with reference to the accompanying figures, in which:
Figure 1 shows a 35 minute STAN® recording in 1^{st} stage of labour in which the fetus is exposed to slowly developing hypoxia;
Figure 2 shows a STAN® recording during the last stages of delivery showing the FHR overacting with an "Alarm reaction" to developing hypoxia;
Figure 3 shows a plot of FHR variation with the identified primary fetal heart rate component superimposed;
Figure 4A shows a plot of the FHR with the identified primary FHR component superimposed;
Figure 4B shows the resulting FHR residual component of FIG 4A;
Figures 5 A and B respectively show the FHR and a plot of the 95^{th} percentile of the FHR residual component in a situation where the fetus is displaying a lack of reactivity;
Figure 6 shows a plot of the frequency distribution of 3-4ms FHR residuals in a fetus showing a lack of reactivity;
Figures 7A and B respectively show the FHR and a plot of the 3-4ms frequency distribution in an instance where the fetus is gradually losing reactivity;
Figures 8A and B respectively show the FHR and a plot of the 3-4ms frequency distribution in an instance where the FHR data alone is ambiguous;
Figure 9 shows a plot of the 95^{th} percentile of the FHR residual component in respect of the fetus recorded in figure 2.
Figure 10 provides a flow chart indicating the different steps in the residual analysis process according to one embodiment of the invention.
Figure 11 is a schematic illustration of the apparatus according to one embodiment of the present invention.

### DETAILED DESCRIPTION

During labour the FHR is monitored for signs of fetal distress (lack of oxygen). Figure 1 shows a 35 minute STAN® recording of a 1st stage of labour. The case illustrates a situation of lack of FHR variability and reactivity (preterminal FHR pattern) that at time of the recording was not recognised in spite of the ST event. The figure also depicts low measurements of "residual" markers of FHR reactivity and variability (Frequency distribution within the 3-4ms range of residual measurements (FD₃₋₄ₘₛ) being <1% and 95^{th} percentile of residual measurements being <3ms).

The FHR 10 is displayed together with uterine activity 12. Here, although the FHR 10 does rise in conjunction with an increase in uterine activity 12 (i.e. contractions), the FHR variation is less than usually expected at this stage. While most experienced obstetricians would be aware that the FHR 10 was abnormal, such a reading would not necessarily trigger other alarms (such as an ST event). Therefore there are occasions when such an abnormal reading would be missed.

Figure 2 shows another example for an abnormal FHR that can sometimes be overlooked upon a visual observation only. This STAN® recording was taken during the final stages delivery and again shows the FHR 20 and uterine activity 22. During this stage of labour it is usual for the FHR 20 to be very erratic and therefore many obstetricians would interpret this reading as normal. Even if other alarms, such a ST events, were raised, confusion over the interpretation of the FHR 20 can lead to delays.

In this case, we have a progressive increase in T/QRS ratio identified by the ST log. The FHR 20 was over-reacting (Alarm reaction) to developing hypoxia. Due to uncertainty over the analysis of the FHR 20, the fetus was born 44 minutes later with signs of oxygen deficiency. Note the marked increase in beat-to-beat variation.

Extreme situations of reduced or increased variability in the heart rate cause problems for interpretation as the above examples show. Using the method described above, the FHR can be separated into a primary component and a residual component.

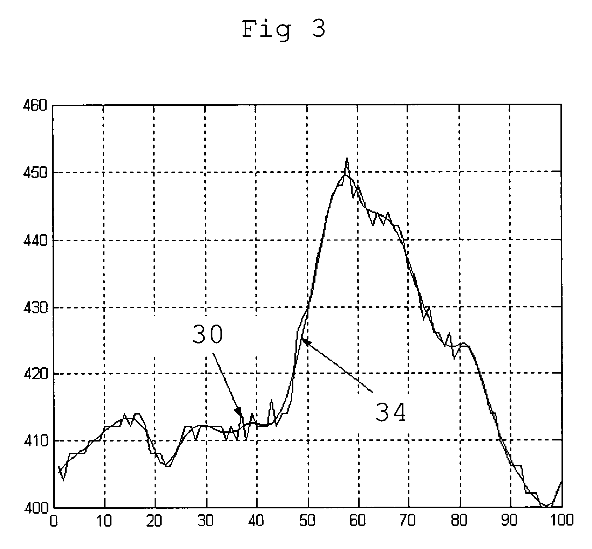

An approximation of the primary FHR component can be calculated by means of polynomial curves fitting. Figure 3 shows a plot of duration (in ms) of consecutive heart beats (RR intervals) over time (in seconds). The polynomial curve fit 34 is shown over the actual RR data 30. Beat-to-beat residual variation corresponds to the difference between the RR data 30 and the curve fit 34 and comprises the FHR residual component. Beat-to-beat variation would correspond to the difference between the RR data and the curve fit.

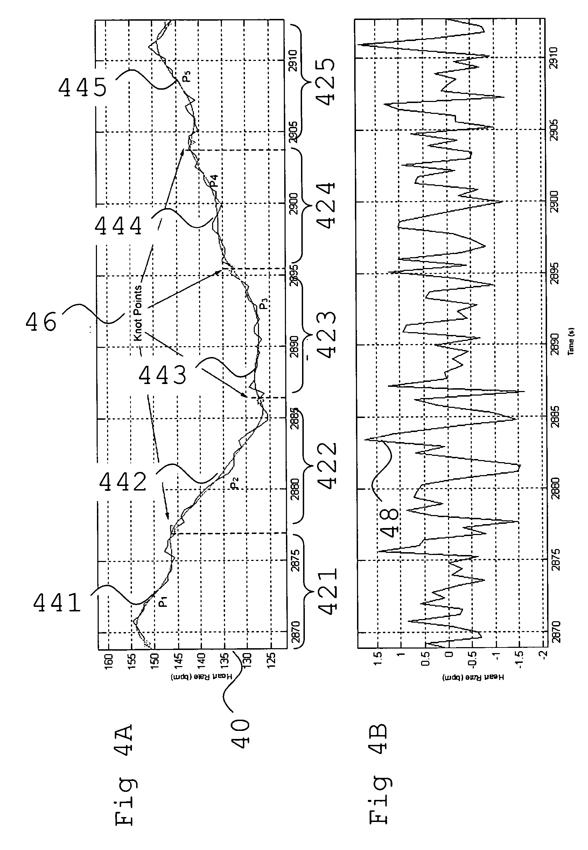

In order to prevent "ringing" in the primary FHR component, the FHR data is divided into small adjacent regions and individual approximations are performed in each region. Figures 4A and 4B show a piecewise polynomial approximation to the HR data. Figure 4A shows a plot of FHR (beats per minute) over time (s). The FHR 40 has been split into 5 adjacent regions 421, 422, 423, 424, 425 of 20 consecutive heart rate samples. A polynomial approximation is carried out in each region 421, 422, 423, 424, 425 to provide individual primary FHR components 441, 442, 443, 444, 445. As the approximations are independent they do not induce "ringing" in the primary FHR component. However, in order to give a smooth continuous curve, it is important to ensure that the primary FHR components 441, 442, 443, 444, 445 align at the boundaries of each region 421, 422, 423, 424, 425. To achieve this, each neighbouring pair of regions shares one heart rate sample. These samples are known as knot points 46. At each knot point 46 the neighbouring polynomial curves are constrained to meet two conditions. Firstly that they have an equal value and secondly that their first derivatives (gradients) are equal. Further constraints can be added so that higher order derivatives must also be equal at the knot points 46, but in this application there is no demonstrated benefit.

Once the primary FHR component has been identified, it is subtracted from the FHR to leave the FHR residual component. Figure 4B shows the FHR residual component 48 which is derived from Figure 4A. Once the FHR residual component 48 has been extracted from the RR or FHR data this can be analysed in a number of ways to monitor the health of the fetus.

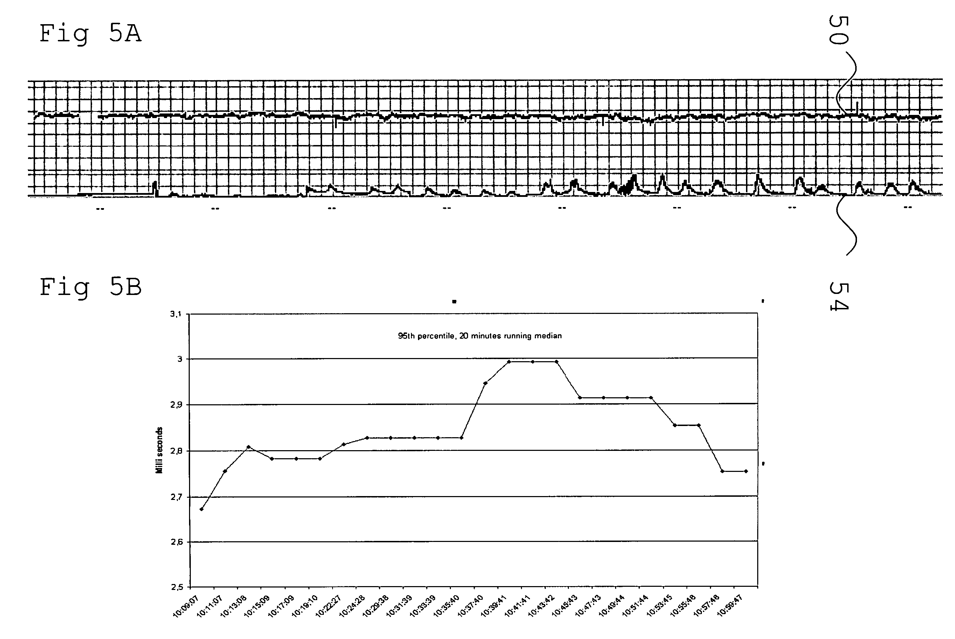

Figure 5A shows the FHR 50 together with the uterine activity 54 in a case of preterminal fetal heart rate pattern recorded during 80 minutes. Figure 5B displays the 95^{th} percentile residual measurements with a 20 minute running median. The 95th percentile shows the level that 95% of the residual component has been below during a certain time period. This reflects changes in the FHR variability and FHR reactivity. From a study of STAN recordings it has been found that a consistent reading of below 3ms, such as that shown in Figure 5B, indicates a lack of reactivity.

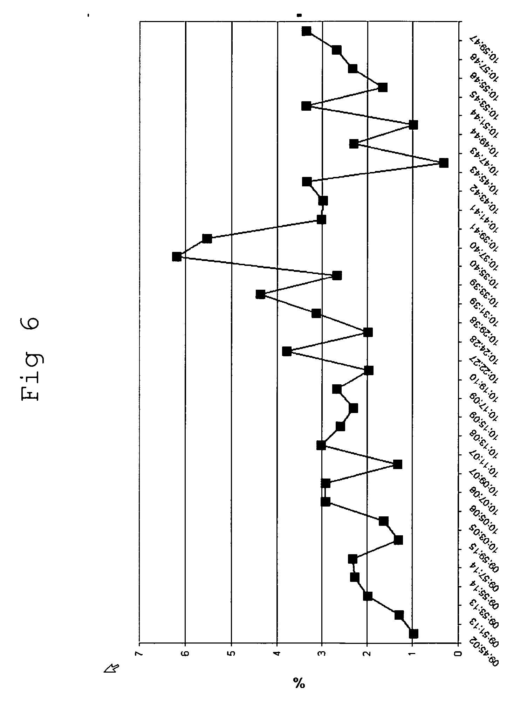

Another area of interest is the 3-4ms frequency distribution. The 3-4ms band has been identified as the highest band which most consistently contains FHR residuals regardless of the fetal heart rate and can thus be used regardless of whether the fetus is in a sleep or active state. It has been found that loss of reactivity can be defined as a situation where the maximum 3-4ms frequency distribution is of <7%. An illustration of this is given in Figure 6 applying the fetal heart rate sequence of Figure 5A.

Some examples, which highlight the benefits of the present invention, are now given.

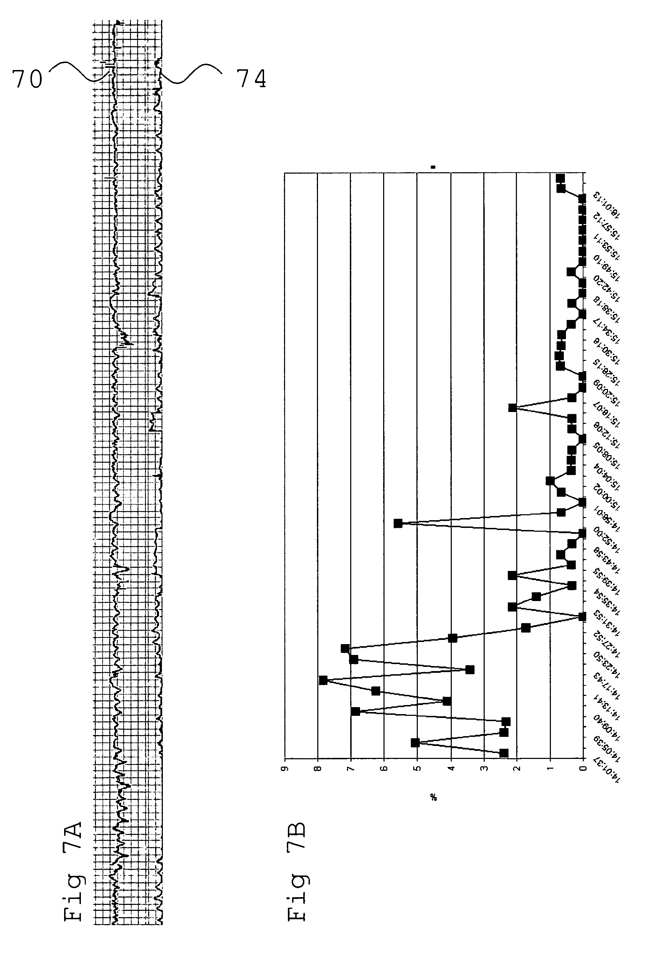

Figure 7A shows the FHR 70 together with the uterine activity 74. In this situation a gradual loss of FHR variation is occurring, which from this data alone can often be missed or at least cause a delay before the loss of reactivity is detected. In this example, the 3-4ms distribution frequency is obtained as described above and this is shown in Figure 7B. It will be seen that in this plot the loss of reactivity is much more apparent and dramatic and therefore easier to detect. With such data available to the obstetrician, diagnosis and action regarding the health of the fetus can be taken more rapidly.

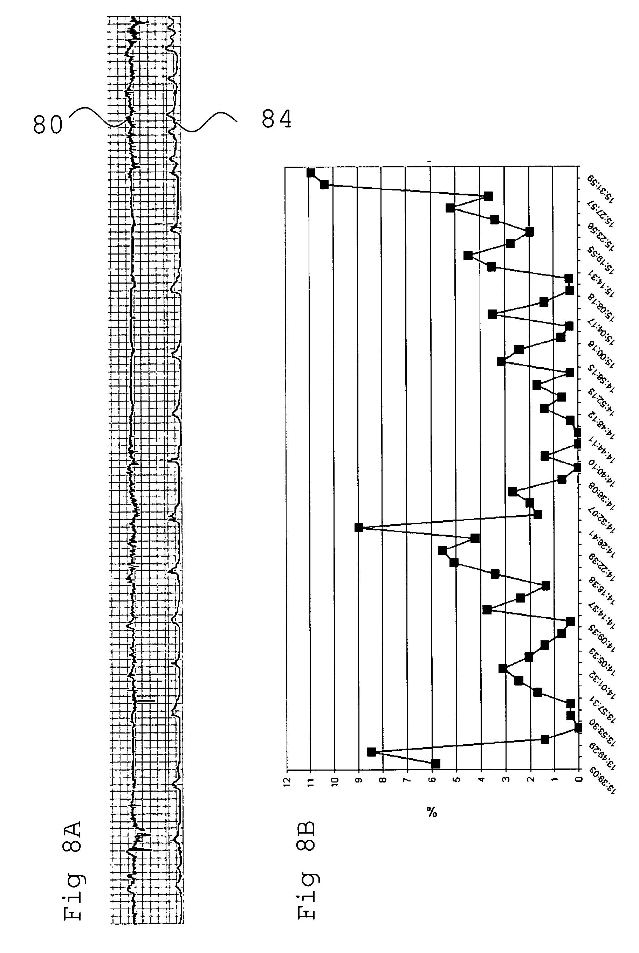

Figure 8A shows another plot of FHR 80, together with uterine activity 84. Here again the FHR variability appears relatively low and from a visual observation may appear to indicate a loss of reactivity. However, from a study of the 3-4ms frequency distribution of the FHR residual component, given in FIG 8B, it is apparent that the residuals are regularly greater than 7%. Therefore, the fetus is healthy and is showing signs of reactivity. Without being able to study this plot, it is possible the obstetrician would make a wrong diagnosis which would lead to unnecessary intervention with the delivery.

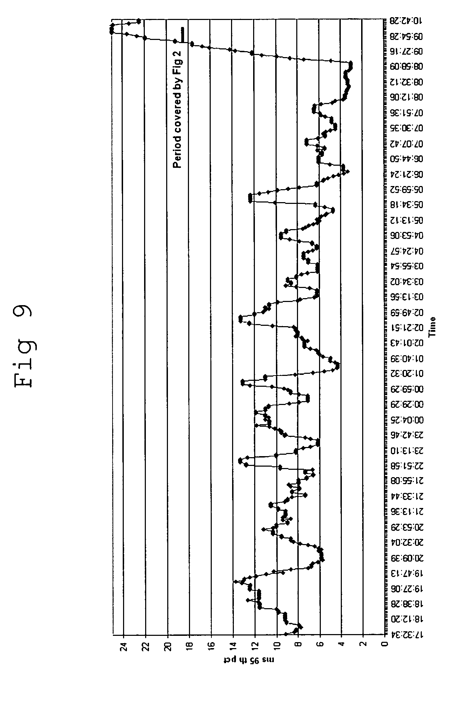

Figure 9 shows the 95th percentile plot of the FHR residual component of FHR 20 recorded in FIG 2. Whereas the FHR readings 20 were indecisive, it is clear from the 95th percentile plot that during the period covered by Figure 2 there is an extreme rise in FHR variability, indicating fetal distress.

The method of the present invention has been applied to stored FHR recordings obtained from thousands of deliveries. Careful study of the FHR residual components in these cases has lead to recommendations for its use according to Table I.

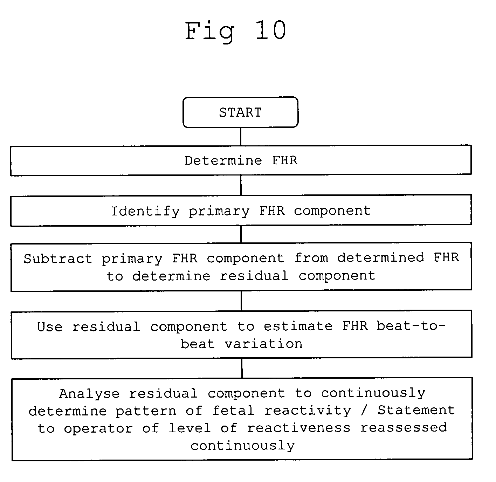

Figure 10 provides a flow chart indicating the different steps in the residual analysis process, according to one emnbodiment of the present invention.

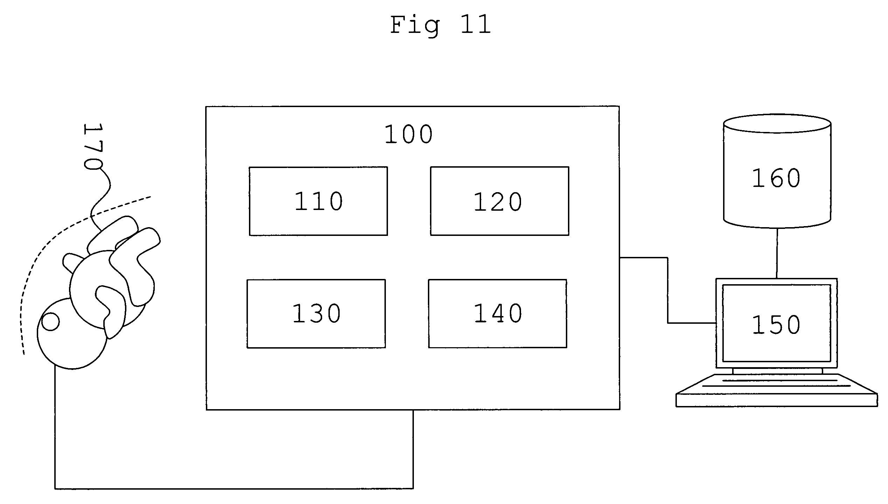

Figure 11 is a schematic illustration of the apparatus 100 according to one embodiment of the present invention, comprising means for determining a fetal heart rate (110), for a fetus (170), means for identifying a primary fetal heart rate component which is required to shift a volume of blood from the heart to the cardiovascular system (120), means for subtracting the primary component from the determined fetal heart rate to determine a residual component (130), means for using said residual component to estimate the fetal heart rate beat-to-beat variation (140), computer means (150), and data storage means (160).

As discussed above, the present invention provides a new and improved way of analysing and monitoring the FHR. This method can be applied during pregnancy and is particularly useful during labour and delivery, when the FHR is non-stationary. The identification of the primary and residual FHR components is a new concept. Careful study of FHR recordings has revealed the most effective methods of applying statistical analysis to the residual FHR component to provide indications of hypoxia.

Specific embodiments of the invention have now been described. However, it should be pointed out that the present invention is not limited to the realizations described above. Several alternatives are possible, as would be apparent for someone skilled in the art. For example, the implementation of the method as discussed above could be accomplished in different ways, such as in especially dedicated hardware or in software, or by a combination ot the two. Further, the means for executing various method steps could be arranged as separate units or entities, but alternatively several method steps could be executed by one single unit. Accordingly, a single unit may perform the functions of several means recited in the claims. Such and other obvious variations must be considered to be part of the present invention, as it is defined in the appended claims.

## Claims

1. An apparatus for fetal monitoring comprising:
a) means for determining a fetal heart rate,
b) means for identifying a primary fetal heart rate component which is required to shift a volume of blood from the heart to the cardiovascular system,
c) means for subtracting the primary component from the determined fetal heart rate to determine a residual component; and
d) means for using said residual component to estimate the fetal heart rate beat-to-beat variation.

2. An apparatus as claimed in claim 1, wherein the primary fetal heart rate component is identified through a polynomial curve fit approximation of the fetal heart rate data.

3. An apparatus as claimed in claim 2, wherein the polynomial curve fit approximation is a 5^{th} order polynomial approximation.

4. An apparatus as claimed in claim 2 or 3, wherein the polynomial approximation is obtained through a least squares approximation.

5. An apparatus as claimed in any preceding claim, wherein the primary fetal heart rate component is determined by:
i) dividing the fetal heart rate data into regions of a predetermined size; and
ii) performing individual polynomial approximations in each region.

6. An apparatus as claimed in claim 5, wherein each polynomial approximation is constrained such that neighbouring polynomial functions align and have equal first derivatives at the region border where they join.

7. An apparatus as claimed in claim 5 or 6, wherein the predetermined size is 20 consecutive heart rate samples.

8. An apparatus as claimed in any preceding claim, wherein the means for using said residual component to estimate the fetal heart rate beat-to-beat variation is adapted to apply statistical tests for analysing the residual component in order to determine the response of the fetus.

9. An apparatus as claimed in claim 8, wherein the statistical test comprises monitoring of a 95^{th} percentile of the fetal heart rate component.

10. An apparatus as claimed in claim 9, wherein if the period of the 95^{th} percentile is consistently below 3ms the fetal heart rate is classed as abnormal and non-reactive.

11. An apparatus as claimed in claim 8, wherein the statistical test comprises monitoring of a short term, e.g. 3-4ms, frequency distribution of the fetal heart rate residual component.

12. An apparatus as claimed in claim 11, wherein if a 3-4ms frequency distribution is less than 7% the fetal heart rate is classed as non-reactive.

13. A method for fetal monitoring comprising the steps of:
a) determining a fetal heart rate,
b) identifying a primary fetal heart rate component which is required to shift a volume of blood from the heart to the cardiovascular system,
c) subtracting the primary component from the determined fetal heart rate to determine a residual component; and
d) using said residual component to estimate the fetal heart rate beat-to-beat variation.

14. A computer program for executing the steps of:
a) determining a fetal heart rate,
b) identifying a primary fetal heart rate component which is required to shift a volume of blood from the heart to the cardiovascular system,
c) subtracting the primary component from the determined fetal heart rate to determine a residual component; and
d) using said residual component to estimate the fetal heart rate beat-to-beat variation.
